# EUROPEAN PATENT SPECIFICATION

(11) **EP 3 944 841 B1**
(45) Date of publication and mention of the grant of the patent: **30.10.2024**
(21) Application number: 21187932.5
(22) Date of filing: 27.07.2021
(51) Int. Cl.: A61F 5/455

(54) **DEVICE FOR AIDING FEMALE URINATION**
VORRICHTUNG ZUR UNTERSTÜTZUNG DES WEIBLICHEN URINIERENS
DISPOSITIF D'AIDE À LA MICTION FÉMININE

(30) Priority: 27.07.2020 IT 202000018091; 27.07.2020 CH 9302020
(43) Date of publication of application: 02.02.2022
(73) Proprietor: Augugliaro, Emanuele Valentino, 6852 Genestrerio (CH)
(72) Inventor: Augugliaro, Emanuele Valentino, 6852 Genestrerio (CH)
(74) Representative: Fabiano, Piero

(56) References cited:
- WO-A1-02/38089
- DE-A1- 4 411 824
- GB-A- 2 002 629
- TW-A- 201 713 285
- US-A- 5 742 948
- US-A1- 2015 223 967
- US-B1- 6 460 200
- US-B2- 7 694 819

## Description

### Field of the invention

The present invention refers to the field of urination devices and in particular to a device for aiding female urination. Even more in detail, the present invention refers to a device for aiding female persons to urinate in an upright position.

### Known art

As known, hygienic conditions of public toilets discourage many women from using these toilets. Sitting in public toilets often dirty and repugnant, not only is psychologically difficult but can be a vehicle of diseases and infections.

It is also well known that, unlike men, outdoor urinating for women, girls or little girls is uncommon and culturally unaccepted because it requires almost total undressing.

In an attempt to solve these problems, several female urination aids - in other words, devices that aid female persons to urinate in an upright position - have been developed.

DE4416094, WO2020086168, US20121491, US5742948, WO02/38089 and US2015/223967 A1, which discloses the preamble of claim 1, describe devices for aiding female urination according to the known art.

The Applicant has observed that the devices for aiding female urination described in the documents above are complex, relatively expensive to manufacture and relatively bulky to transport.

The Applicant has therefore found the need to provide a new type of device for aiding female urination that can be easily transported also in the pocket of a garment, such as trousers, that is simple and intuitive to use, that can be manufactured simply and economically.

### Summary of the invention

Therefore the invention, in a first aspect thereof, concerns a device for aiding female urination, according to claim 1.

This way, the device for aiding female urination can be folded on itself and flattened to be comfortably transported also in the trousers' pocket.

The flap is integral with the tubular body and is joined thereto by means of a folding line which extends in a direction substantially transverse to the longitudinal axis.

Conveniently, the flap has an extension along the longitudinal axis between 6 cm and 12 cm.

Advantageously, the flap has a curvilinear shape comprising, in a planar configuration of the tubular body, two longitudinal curvilinear lengths extending substantially parallel to the longitudinal axis and a curvilinear connecting length positioned between said two longitudinal curvilinear lengths.

Preferably, the two longitudinal curvilinear lengths are placed at a minimum distance in the transverse direction greater than 1.5 cm.

Advantageously, the proximal end has, in an operating configuration, a diameter greater than the diameter of said distal end.

Conveniently, the proximal end has, in an operating configuration, a diameter greater than 4 cm.

Preferably, the tubular body in operating configuration tapers from the proximal end toward the distal end.

Conveniently, at least the tubular body is made of biodegradable and/or compostable material.

Advantageously, at least the tubular body is made of paper material.

Further characteristics and advantages of the invention will become more apparent from the detailed description of some preferred, but not exclusive, embodiments of a device for aiding female urination according to the present invention.

### Brief description of the drawings

Such description will be set forth hereinafter with reference to the appended drawings provided for indicative, and therefore non-limiting, purpose only, wherein:
- figure 1 shows a schematic view of the device for aiding female urination according to the present invention with the tubular body in a planar configuration and the flap folded;

figure 2 is a schematic view of the device for aiding female urination according to the present invention with the tubular body in a planar configuration and the flap folded;
figure 3 shows a schematic view of the device for aiding female urination according to the present invention with the tubular body in the operating configuration and the flap rotated, also in the operating position;
figure 4 shows a schematic view of the device for aiding female urination according to the present invention in use and resting on the body of a user.

### Detailed description of embodiments of the invention

With reference to the figures, a device for aiding female urination according to the present invention is depicted with the reference numeral 1.

The device for aiding female urination 1 comprises a tubular body 2 which extends along a longitudinal axis X-X, between a proximal end 3 configured to rest in use on the user's body at the female urination organ and a distal end 6 at some distance from the proximal end.

The tubular element 2 has at least one side wall 4 extending between the distal end 6 and the proximal end 3.

Preferably, the tubular body 2 has an extension along the longitudinal axis X-X of less than 15 cm, even more preferably less than 13 cm.

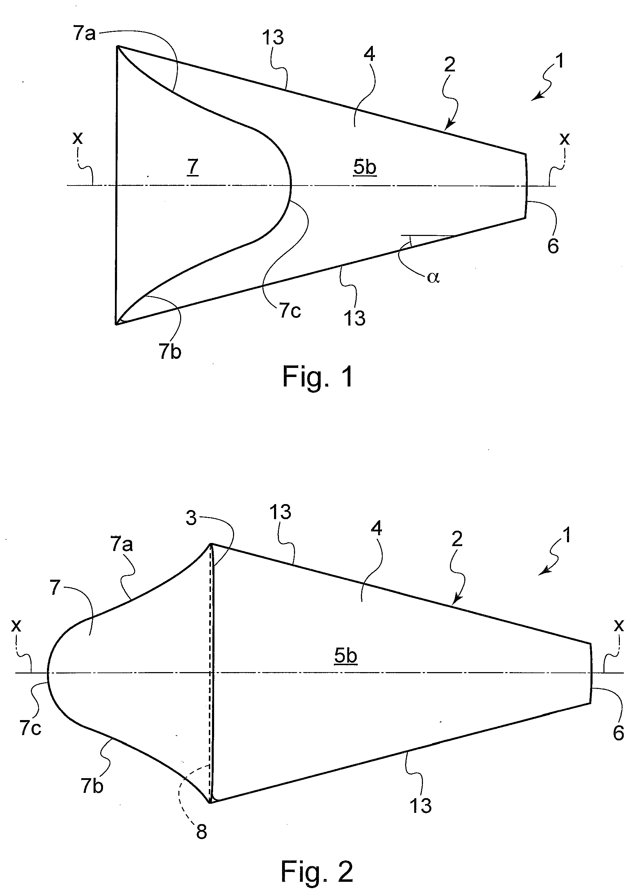

The tubular body 2 is formed so as to be able to switch from a planar configuration shown in Figures 1 and 2, in which its side wall 4 is folded on itself to form two opposite portions 5a, 5b: a lower opposite portion 5a and an upper opposite portion 5b, respectively, and an operating configuration, in which its side wall 4 is arranged around a circumference 16 so that the lower opposite portion 5a and the upper opposite portion 5b are arranged according to arcs of said circumference 16.

Preferably, the lower opposite portion 5a and the upper opposite portion 5b are substantially the same in shape and size.

The lower opposite portion 5a and the upper opposite portion 5b are in a planar configuration substantially, except for a vertex, of triangular shape.

In a planar configuration, the upper opposite portion 5b is overlapped on the lower opposite portion 5a, so as to cover it entirely.

In other words, in a planar configuration, the upper opposite portion 5b substantially perfectly overlaps the lower opposite portion 5a so that none of the two opposite portions 5a, 5b projects with respect to the perimeter of the remaining opposite portion.

In the embodiment shown in the figures, the upper 5b and lower 5b opposite portions are joined together through two folding lines 13.

In a planar configuration, the folding lines 13 of the tubular body 2 extend opposite with respect to the longitudinal axis X-X so as to form with the latter an angle α between 0° and 60°, preferably between 5° and 45°.

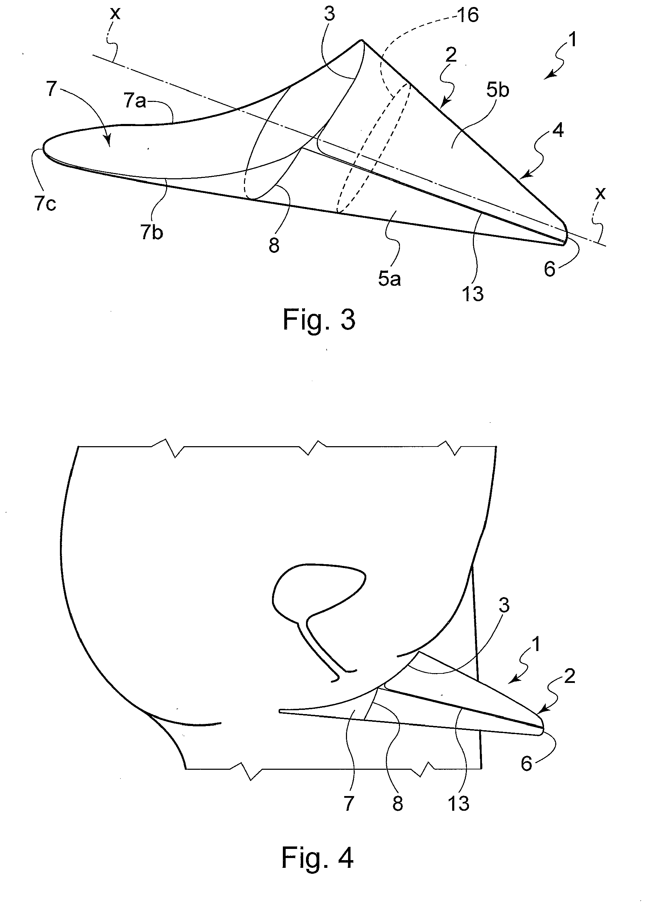

In a planar configuration, by exerting a slight pressure at the folding lines 13, the upper opposite portion 5b and the lower opposite portion 5a bend, making the tubular body 3 assume its operating configuration shown in figures 3 and 4.

Preferably, in order to help in conveying the urine being dispensed away from the user's body, the tubular body 2, in an operating configuration, is tapered in the direction of the distal end 6.

In the embodiment shown in the figures, the proximal end 3 has in an operating configuration a diameter greater than the diameter of the distal end 6. Preferably, the proximal end 3 has in an operating configuration a diameter greater than or equal to twice the diameter of the distal end 6.

The proximal end 3 has in an operating configuration a diameter greater than 4 cm, preferably greater than 5 cm.

Preferably, the tubular body 2 is made of biodegradable and/or compostable material, even more preferably the tubular body 2 is made of biodegradable and/or compostable paper material.

Preferably, the tubular body 2 extends along the axis X-X for a length of less than 15 cm, preferably less than 12 cm.

According to an important aspect of the present invention, the device 1 for aiding female urination has a flap 7.

The flap extends from the proximal end 3, in particular from its perimeter edge.

The flap 7 is configured to switch from a rest position shown in figure 1, in which the flap 7 is at least partially folded and overlapped on one of the upper opposite portion 5b and the lower opposite portion 5a, to an operating position, in which the flap 7 is rotated with respect to the rest position and extends along the extension direction of one of the upper opposite portion 5b and the lower opposite portion 5a.

In the embodiment shown in the figures, the flap 7 is integral with the lower opposite portion 5a, and therefore rotates from the rest position shown in figure 1, in which it is folded and overlapped on the upper opposite portion 5b, to the operating position shown in figure 2,3,4, in which it extends along the extension direction of the lower opposite portion 5a.

In the embodiment shown in the figures, when the flap 7 switches from the rest position to the operating position, it rotates by at least 135°.

Preferably, in the embodiment shown in the figures, when switching from the rest position to the operating position, the flap 7 rotates substantially by about 180°. In order to switch from a rest position to an operating position, the flap 7 rotates only with respect to the folding line 8.

In other words, there are no other rotations of the flap 7 to bring it from the rest position to the operating position.

Preferably, the flap 7 is integral with the tubular body 3 and is joined thereto by means of a folding line 8 which extends in a direction substantially transverse to the longitudinal axis X-X.

Preferably, a folding line 8 extending in a direction substantially transverse to the longitudinal axis X-X across the width of the flap 7. In particular, the folding line 8 is arranged at a length of the perimeter edge of the proximal end 3.

In the embodiment shown in the figures, the flap 7 in the rest position is flat and has a curvilinear shape comprising two longitudinal curvilinear lengths 7a, 7b extending substantially in the direction of the longitudinal axis X-X and a curvilinear connecting length 7c placed between the two longitudinal curvilinear lengths 7a, 7b to join them together.

Preferably, the two longitudinal curvilinear lengths 7a, 7b are placed at a minimum distance in the transverse direction greater than 1.5 cm.

The two longitudinal curvilinear lengths 7a, 7b are placed at a maximum distance in the transverse direction of less than 15 cm, preferably less than 12 cm.

The two longitudinal curvilinear lengths 7a, 7b are positioned at a maximum distance in the transverse direction at the folding line 8.

Proceeding towards the curvilinear connecting length 7c, the two longitudinal curvilinear lengths 7a, 7b are placed at a decreasing distance.

The curvilinear shape of the flap 7 is designed so as to support the ergonomics of the inside of the user's thigh in order to be as comfortable as possible during use, when the flap 7 is rotated and between the user's thighs, as shown in Figure 4.

Advantageously, the flap 7 has an extension along the longitudinal axis X-X between 6 cm and 12 cm.

Preferably between 3 cm and 8 cm, this way, once folded, the flap 7 does not project in the longitudinal direction with respect to the upper or lower opposite portion, which will therefore represent the maximum possible encumbrance of the device 1 in the longitudinal direction according to the present invention.

In order to adapt effectively to the female body by not allowing drips from the rear, the flap 7 has an extension along the longitudinal axis X-X greater than 30% of the extension of the tubular body 2 along the longitudinal axis.

Preferably, the flap 7 has an extension along the longitudinal axis X-X greater than 35% of the extension of the tubular body 2 along the longitudinal axis.

The flap 7 has a width measured in the transverse direction, i.e. substantially orthogonal to the longitudinal axis X-X, smaller than the width in the planar configuration of the tubular body 2, in this way once folded, the flap 7 does not project in a transverse direction with respect to the upper or lower opposite portion, which will therefore represent in a transverse direction the maximum possible encumbrance of the device 1 according to the present invention.

In the embodiment shown in the figures, the flap 7 being made of a single piece with the tubular body 2, it is also made of biodegradable and/or compostable material. Even more preferably, the flap 7 is made of biodegradable and/or compostable paper material.

In other words, the entire device for aiding female urination is made of biodegradable and/or compostable paper material, thus making the device single-use and disposable.

This device 1 for aiding female urination has the following advantages:
- limited encumbrance and consequent ease to carry, even in a garment pocket, such as the back pocket of j eans-type trousers;
- ease of disposal and low environmental impact, being made of biodegradable and/or compostable material;
- ease of use for the ergonomics of the shape of the flap;
- ease and cost-effectiveness of production;
- extreme hygiene, being substantially single-use.

Several changes can be made to the embodiments described in detail, while remaining within the scope of the invention protection, defined by the following claims.

## Claims

1. Device (1) for aiding female urination, comprising:
- a tubular body (2) extending along a longitudinal axis (X-X), between a proximal end (3) configured to rest during use on the female body portion at the genital organ, and a distal end (6);
- said tubular element (2) having at least one side wall extending between said distal end (6) and said proximal end (3);
- said tubular body (2) having an extension along the longitudinal axis (X-X) of less than 15 cm;
- said tubular body (2) being configured to switch from a planar configuration, in which the side wall (4) is folded on itself to form at least two opposite portions (5a,5b), respectively a lower opposite portion (5a) and an upper opposite portion (5b), and an operating configuration, in which the side wall (4) is arranged substantially around a circumference in such a way that said lower opposite portion (5a) and said upper opposite portion (5b) are arranged to follow arcs of said circumference;
**characterised by** comprising a flap (7) integral with said tubular body (3) and joined thereto by means of a folding line (8), which extends in a direction substantially transverse to said longitudinal axis (X-X) said flap being configured to switch from a rest position, in which said flap (7) is at least partially folded and overlapped on one of said upper opposite portion (5b) and said lower opposite portion (5a) to an operating position, in which said flap (7) is rotated with respect to said rest position and extends along the extension direction of one of said upper opposite portion (5b) and said lower opposite portion (5a); during the switching from the rest position to the operating position said flap (7) rotates by at least 135°; in order to switch from a rest position to an operating position, the flap (7) rotates only with respect to the folding line (8).

2. Device (1) for aiding female urination according to claim 1, **characterised in that** an extension of said flap (7) along said longitudinal axis (X-X) is between 6 cm and 12 cm.

3. Device (1) for aiding female urination according to claim 1, **characterised in that** said flap (7) has a curvilinear shape comprising, in planar configuration of the tubular body (2), two longitudinal curvilinear lengths (7a,7b) extending substantially parallel to said longitudinal axis (X-X) and a curvilinear connecting length (7c) placed between said two longitudinal curvilinear lengths (7a,7b).

4. Device (1) for aiding female urination according to claim 1, **characterised in that** said two longitudinal curvilinear lengths (7a,7b) are placed, in the transverse direction, at a minimum distance greater than 1.5 cm.

5. Device (1) for aiding female urination according to claim 1, **characterised in that** said proximal end (3) has, in the operating configuration, a diameter greater than the diameter of said distal end (6).

6. Device (1) for aiding female urination according to claim 1, **characterised in that** said proximal end (6) has, in the operating configuration, a diameter greater than 4 cm.

7. Device (1) for aiding female urination according to claim 1, **characterised in that** said tubular body (2) in the operating configuration tapers from said proximal end (3) to said distal end (6).

8. Device (1) for aiding female urination according to claim 1, **characterised in that** at least said tubular body (2) is made of biodegradable and/or compostable material.

9. Device (1) for aiding female urination according to any one of the preceding claims, **characterised in that** at least said tubular body (2) is made of paper material.

10. Device (1) for aiding female urination according to any one of the preceding claims, **characterized in that** the flap (7) has an extension along the longitudinal axis X-X greater than 30% of the extension along the longitudinal axis of the tubular body (2).

## Patentansprüche

1. Vorrichtung (1) zur Hilfe beim weiblichen Urinieren, umfassend:
- einen röhrenförmigen Körper (2), der sich entlang einer Längsachse (X-X) zwischen einem, während der Benutzung auf dem weiblichen Körperteil am Genitalorgan aufliegenden, proximalen Ende (3) und einem distalen Ende (6) erstreckt;
- wobei das rohrförmige Element (2) mindestens eine zwischen dem distalen Ende (6) und dem proximalen Ende (3) verlaufende Seitenwand aufweist;
- wobei der röhrenförmige Körper (2) eine Erstreckung entlang der Längsachse (X-X) von weniger als 15 cm aufweist;
- wobei der rohrförmige Körper (2) derart gestaltet ist, dass er zwischen einer planen Gestalt, in der die Seitenwand (4) auf sich selbst gefaltet ist, zur Bildung von mindestens zwei gegenüberliegenden Abschnitten (5a, 5b), d.h. einem unteren gegenüberliegenden Abschnitt (5a) bzw. einem oberen gegenüberliegenden Abschnitt (5b), und einer operativen Gestalt umschaltbar ist, in der die Seitenwand (4) im Wesentlichen um einen Umfang herum so angeordnet ist, dass der untere gegenüberliegende Abschnitt (5a) und der obere gegenüberliegende Abschnitt (5b) den Bögen des besagten Umfangs folgend angeordnet sind;
**gekennzeichnet durch** Umfassen einer einstückig mit dem rohrförmigen Körper (3) ausgebildeten Klappe (7), welche mit diesem mittels einer sich in einer Richtung im Wesentlichen quer zur Längsachse (X-X) erstreckenden Faltlinie (8) verbunden ist, wobei die Klappe so ausgestaltet ist, dass sie von einer Ruhestellung, in der die Klappe (7) zumindest teilweise gefaltet und auf einem des oberen gegenüberliegenden Abschnitts (5b) und des unteren gegenüberliegenden Abschnitts (5a) überlappt ist, in eine Betriebsstellung umschaltbar ist, in der die Klappe (7) in Bezug auf die Ruhestellung verschwenkt ist und sich entlang der Erstreckungsrichtung von einem des oberen gegenüberliegenden Abschnitts (5b) und des unteren gegenüberliegenden Abschnitts (5a) erstreckt; wobei die Klappe (7) während des Umschaltens von der Ruheposition in die Betriebsposition um mindestens 135° schwenkt; und die Klappe (7) zum Umschalten von einer Ruheposition in eine Betriebsposition nur in Bezug auf die Faltlinie (8) schwenkt.

2. Vorrichtung (1) zur Hilfe beim weiblichen Urinieren nach Anspruch 1, **dadurch gekennzeichnet, dass** eine Erstreckung der Klappe (7) entlang der Längsachse (X-X) zwischen 6 cm und 12 cm beträgt.

3. Vorrichtung (1) zur Hilfe beim weiblichen Urinieren nach Anspruch 1, **dadurch gekennzeichnet, dass** die Klappe (7) eine kurvenförmige Form aufweist, die in der planen Gestalt des röhrenförmigen Körpers (2) zwei sich im Wesentlichen parallel zu der Längsachse (X-X) erstreckende kurvenförmige Längsabschnitte (7a, 7b) und einen zwischen den beiden kurvenförmigen Längsabschnitten (7a, 7b) angeordneten kurvenförmigen Verbindungsabschnitt (7c) umfasst.

4. Vorrichtung (1) zur Hilfe beim weiblichen Urinieren nach Anspruch 1, **dadurch gekennzeichnet, dass** die beiden kurvenförmigen Längsabschnitte (7a, 7b) in Querrichtung mit einem Mindestabstand von mehr als 1,5 cm angeordnet sind.

5. Vorrichtung (1) zur Hilfe beim weiblichen Urinieren nach Anspruch 1, **dadurch gekennzeichnet, dass** das proximale Ende (3) in der operativen Gestalt einen Durchmesser aufweist, der größer als der Durchmesser des distalen Endes (6) ist.

6. Vorrichtung (1) zur Hilfe beim weiblichen Urinieren nach Anspruch 1, **dadurch gekennzeichnet, dass** das proximale Ende (6) in der operativen Gestalt einen Durchmesser von mehr als 4 cm hat.

7. Vorrichtung (1) zur Hilfe beim weiblichen Urinieren nach Anspruch 1, **dadurch gekennzeichnet, dass** sich der rohrförmige Körper (2) in der operativen Gestalt von dem proximalen Ende (3) zu dem distalen Ende (6) verjüngt.

8. Vorrichtung (1) zur Hilfe beim weiblichen Urinieren nach Anspruch 1, **dadurch gekennzeichnet, dass** zumindest der röhrenförmige Körper (2) aus biologisch abbaubarem und/oder kompostierbarem Material hergestellt ist.

9. Vorrichtung (1) zur Hilfe beim weiblichen Urinieren nach einem der vorhergehenden Ansprüche, **dadurch gekennzeichnet, dass** zumindest der röhrenförmige Körper (2) aus Papiermaterial hergestellt ist.

10. Vorrichtung (1) zur Hilfe beim weiblichen Urinieren nach einem der vorhergehenden Ansprüche, **dadurch gekennzeichnet, dass** die Klappe (7) eine Erstreckung entlang der Längsachse X-X von mehr als 30% der Erstreckung entlang der Längsachse des rohrförmigen Körpers (2) aufweist.

## Revendications

1. Dispositif (1) d'aide à la miction féminine, comprenant:
- un corps tubulaire (2) s'étendant le long d'un axe longitudinal (X-X), entre une extrémité proximale (3) configurée pour reposer lors de l'utilisation sur la portion du corps féminin au niveau de l'organe génital, et une extrémité distale (6);
- ledit élément tubulaire (2) ayant au moins une paroi latérale s'étendant entre ladite extrémité distale (6) et ladite extrémité proximale (3);
- ledit corps tubulaire (2) ayant une extension le long de l'axe longitudinal (X-X) inférieure à 15 cm;
- ledit corps tubulaire (2) étant configuré pour passer d'une configuration plane, dans laquelle la paroi latérale (4) est repliée sur elle-même pour former au moins deux portions opposées (5a,5b), respectivement une portion opposée inférieure (5a) et une portion opposée supérieure (5b), à une configuration de fonctionnement, dans laquelle la paroi latérale (4) est disposée sensiblement autour d'une circonférence de sorte à ce que ladite portion opposée inférieure (5a) et ladite portion opposée supérieure (5b) soient disposées pour suivre des arcs de ladite circonférence;
**caractérisé en ce qu'**il comprend un rabat (7) solidaire dudit corps tubulaire (3) et relié au même au moyen d'une ligne de pliage (8), qui s'étend dans une direction sensiblement transversale audit axe longitudinal (X-X), ledit rabat étant configuré pour passer d'une position de repos, dans laquelle ledit rabat (7) est au moins partiellement plié et chevauché sur l'une de ladite portion opposée supérieure (5b) et ladite portion opposée inférieure (5a), à une position de fonctionnement, dans laquelle ledit rabat (7) est tourné par rapport à ladite position de repos et s'étend le long de la direction d'extension de l'une de ladite portion opposée supérieure (5b) et ladite portion opposée inférieure (5a); lors du passage de la position de repos à la position de fonctionnement, ledit rabat (7) tourne d'au moins 135°; afin de passer d'une position de repos à une position de fonctionnement, le rabat (7) tourne uniquement par rapport à la ligne de pliage (8).

2. Dispositif (1) d'aide à la miction féminine selon la revendication 1, **caractérisé en ce qu'**une extension dudit rabat (7) le long dudit axe longitudinal (X-X) est comprise entre 6 cm et 12 cm.

3. Dispositif (1) d'aide à la miction féminine selon la revendication 1, **caractérisé en ce que** ledit rabat (7) a une forme curviligne comprenant, dans une configuration plane du corps tubulaire (2), deux longueurs curvilignes longitudinales (7a,7b) s'étendant sensiblement parallèlement audit axe longitudinal (X-X) et une longueur de connexion curviligne (7c) placée entre lesdites deux longueurs curvilignes longitudinales (7a,7b).

4. Dispositif (1) d'aide à la miction féminine selon la revendication 1, **caractérisé en ce que** lesdites deux longueurs curvilignes longitudinales (7a,7b) sont placées, dans la direction transversale, à une distance minimum supérieure à 1,5 cm.

5. Dispositif (1) d'aide à la miction féminine selon la revendication 1, **caractérisé en ce que** ladite extrémité proximale (3) a, dans la configuration de fonctionnement, un diamètre supérieur au diamètre de ladite extrémité distale (6).

6. Dispositif (1) d'aide à la miction féminine selon la revendication 1, **caractérisé en ce que** ladite extrémité proximale (6) a, dans la configuration de fonctionnement, un diamètre supérieur à 4 cm.

7. Dispositif (1) d'aide à la miction féminine selon la revendication 1, **caractérisé en ce que** ledit corps tubulaire (2) se rétrécit de ladite extrémité proximale (3) à ladite extrémité distale (6) dans la configuration de fonctionnement.

8. Dispositif (1) d'aide à la miction féminine selon la revendication 1, **caractérisé en ce qu'**au moins ledit corps tubulaire (2) est réalisé en un matériau biodégradable et/ou compostable.

9. Dispositif (1) d'aide à la miction féminine selon l'une quelconque des revendications précédentes, **caractérisé en ce qu'**au moins ledit corps tubulaire (2) est réalisé en un matériau papier.

10. Dispositif (1) d'aide à la miction féminine selon l'une quelconque des revendications précédentes, **caractérisé en ce que** le rabat (7) a une extension le long de l'axe longitudinal X-X supérieure à 30% de l'extension le long de l'axe longitudinal du corps tubulaire (2).
